# EUROPÄISCHE PATENTSCHRIFT

(11) **EP 1 148 846 B1**
(45) Veröffentlichungstag und Bekanntmachung des Hinweises auf die Patenterteilung: **17.08.2005**
(21) Anmeldenummer: 99967920.2
(22) Anmeldetag: 28.12.1999
(51) Int. Cl.: A61F 2/16

(54) **AKKOMMODATIVE INTRAOKULARLINSE**
ACCOMMODATIVE INTRAOCULAR LENS
LENTILLE INTRAOCULAIRE APTE A L'ACCOMMODATION

(30) Priorität: 01.02.1999 DE 19904441
(43) Veröffentlichungstag der Anmeldung: 31.10.2001
(73) Patentinhaber: Preussner, Paul Rolf, Dr., 55131 Mainz (DE)
(72) Erfinder: Preussner, Paul Rolf, Dr., 55131 Mainz (DE)
(86) Internationale Anmeldenummer: PCT/DE1999/004123
(87) Internationale Veröffentlichungsnummer: WO 2000/045745

(56) Entgegenhaltungen:
- US-A- 4 298 996
- US-A- 5 171 266
- US-A- 5 562 731
- US-A- 5 800 533

## Beschreibung

### Technisches Gebiet

Bei der Erfindung handelt es sich um eine Intraokularlinse mit zugehörigen Hilfsvorrichtungen, mit der ein Patient durch bzw. nach Operation der natürlichen Augenlinse wieder die Fähigkeit zur optischen Naheinstellung (Akkommodation) erlangt.

### Stand der Technik

Intraokularlinsen, wie sie dem Stand der Technik entsprechen, erlauben normalerweise nur ein scharfes Sehen in genau einer Entfernung. Daneben sind seit einigen Jahren Intraokularlinsen bekannt, die zwei oder mehrere Brennweiten aufweisen ("Multifokallinsen") und so ein scharfes Sehen in mehreren Entfernungen zulassen. Da dabei mehrere Bilder unscharf überlagert werden, ist mit diesen Linsen der Kontrast deutlich schlechter als mit einer Monofokallinse. Auch wird nur in bestimmten Entfernungen scharf gesehen, der Zwischenbereich ist deutlich unschärfer. Ein Sehen wie bei natürlicher Akkommodation ist mit diesen Linsen also nicht erreichbar.

Einen gedanklichen Zwischenschritt in Richtung Akkommodation stellen Systeme dar, bei denen die Brechkraft des intraokularen Implantates durch äußere Maßnahmen adjustiert werden kann. Die optischen Elemente können dabei beispielsweise durch Änderung der Kopfhaltung mittels Schwerkraft oder durch magnetische Kräfte (US5326347A, US5593437A) auf der optischen Achse verschoben werden. Im letzteren Fall ist auf der Intraokularlinse eine magnetische Schicht aufgebracht, und die Linse wird durch einen adjustierbaren Magneten, der vor dem Auge in einer Art Brille sitzt, bewegt. In US5800533A wird die Verschiebung auf der optischen Achse mittels eines Schraubgewindes vorgenommen, wobei der Schraubvorgang mit magnetischen Werkzeugen von außerhalb des Auges vorgenommen wird.

Um eine vom Patienten als "natürlich " empfundene Akkommodationsfähigkeit nach Entfernung des Inneren der getrübten, eigenen Augenlinse (Katarakt, grauer Star) wiederherzustellen, genügt die oben beschriebene Adjustierbarkeit nicht. Statt dessen muß die Brechkraft der implantierten Kunstlinse beispielsweise über ihre Form oder durch Verschiebung auf der optischen Achse oder durch Verschiebung mehrerer optischer Elemente gegeneinander verändert werden, wenn sich der Ziliarmuskel kontrahiert. Nur dieser Vorgang soll im folgenden unter "Akkommodation" verstanden werden. Zu seiner Realisierung sind mehrere Ansätze bekannt geworden. In den Kapselsack kann ein mit einer Flüssigkeit füllbarer Ballon eingesetzt werden (EP0493090A1). Zwei optisch wirksame Komponenten können durch Flüssigkeitsdruck gegeneinander verschoben werden (EP0356050B1). Optische Komponenten werden durch Federdruck verschoben (EP0337390A2). Außerdem sind Intraokularlinsen kommerziell verfügbar, deren äußerer Teil (Haptik) als federndes Scharnier ausgebildet ist, so daß eine radiale Kontraktion in eine axiale Verschiebung umgelenkt wird (WO9615734A2).

Keiner der genannten Ansätze hat bisher eine praktische Bedeutung erlangen können. Der Grund ist für alle prinzipiell der gleiche: Nach Entfernung des Linseninneren kommt es zu einer individuell stark unterschiedlichen Schrumpfung und Verhärtung des Kapselsackes. Der Kapselsack kann somit nicht als ein in seinen mechanischen Parametern berechenbares Element eines wie auch immer gearteten Bewegungsvorganges verwendet werden. Außerdem weist auch die Federkraft der Zonulafasern, über die der Kapselsack elastisch am Ziliarmuskel aufgehängt ist, erhebliche interindividuelle Schwankungen auf. Schließlich ist bei einem großen Teil der Patienten nach Kataraktoperation damit zu rechnen, daß es zu einer Trübung der hinteren Linsenkapsel kommt (Nachstar). Dieser getrübte Teil muß dann entfernt werden, beispielsweise mit einem Laser, was die mechanischen Eigenschaften des Kapselsackes erneut drastisch ändert.

Ein akkommodatives Intraokulalinsensystem gemäß dem Oberbegriff des Anspruchs 1 ist aus dem Dokument US-A-5 171 266 bekannt.

Aufgabe der vorliegenden Erfindung ist es, eine Intraokularlinse verfügbar zu machen, die ihre Position in axialer Richtung auf Kontraktion des Ziliarmuskels verändert. Die postoperative Schrumpfung des Kapselsackes, die Variation in der Federkraft der Zonulafasern und eine spätere Entfernung des hinteren, zentralen Teils der Kapsel (Kapsulotomie) sollen die Funktion des Akkommodationsvorganges nicht wesentlich beeinflussen.

### Kurze Darstellung der Erfindung

Die Aufgabe wird erfindungsgemäß entsprechend Anspruch 1 gelöst. Das intraokulare, in bekannter Weise in den Kapselsack einzusetzende Implantat enthält zentral eine Kunstlinse in bekannter Technik und in seinem peripheren Teil Permanentmagnete, die auf einem Kreis mit der optischen Achse des Auges als Kreisnormale angeordnet sind (im folgenden "innere Magnete" genannt). Diesen inneren Magneten gegenüber sind auf einem weiter hinten, außen liegenden Kreis, dessen Kreisnormale wieder die optische Achse des Auges ist, ebenfalls Permanentmagnete an der Sklera befestigt (im folgenden "äußere Magnete" genannt). Innere und äußere Magnete sind so gepolt, daß sie sich gegenseitig abstoßen, und geometrisch so ausgerichtet, daß diese Abstoßung eine nach vorne gerichtete Kraft auf den Kapselsack und damit auf die darin befindliche Intraokularlinse ausübt.

### Bevorzugte Ausführung und Ausführungsalternativen

In seiner bevorzugten Ausführung besteht das in den Kapselsack einzusetzende Implantat aus einer beliebigen Hinterkapsellinse mit zentraler Optik und vorzugsweise Schleifenhaptik in bekannter und bewährter Technik sowie einem Kapselspannring ebenfalls in bekannter und bewährter Technik.

In den Kapselspannring sind die inneren Magnete eingegossen. Die Magnete müssen vollständig von biokompatiblem Material umgeben sein. Um die Dichte des Ringes zu reduzieren, kann der Ring zwischen den inneren Magneten Hohlräume aufweisen. Die äußeren Magnete können als Stabmagnete, umgeben von biokompatiblem Material, ausgeführt sein, wobei bereits vorkonfektionierte Ösen, Kerben oder Fäden die genaue Positionierung und das Annähen erleichtern. Die äußeren Magnete werden bevorzugt von außen auf der Sklera unter der Bindehaut angenäht. Prinzipiell können sie auch im Augeninneren im Bereich der pars plana befestigt werden. Von den magnetischen Kräften her ist letzteres günstiger, das Operationsrisiko ist aber deutlich höher.

In einer anderen Ausführung der Erfindung werden die inneren Magnete in die Haptik der Intraokuiarlinse integriert. Bei einer Schleifenhaptik, beispielsweise mit C- oder J-Schleifen in bekannter Technik, können die Magnete in die Enden der Schleifen eingesetzt sein. Vorteilhaft an dieser Ausführung ist die Möglichkeit, diese Linsen als Faltlinsen in bekannter Technik ausführen zu können. Allerdings sind dabei nur zwei innere Magnete möglich, was die magnetische Kraft limitiert. Bei einer Plattenhaptik können im Außenbereich mehrere Magnete angebracht sein, bei dieser ist aber eine größere Asymmetrie in der Schrumpfung des Kapselsackes als Nachteil bekannt.

## Patentansprüche

1. Akkommodatives Intraokularlinsensystem mit
• einer Intraokularlinse in bekannter Technik zum Einsetzen in das Innere des Kapselsackes,
• inneren Permanentmagneten, die im Inneren des Kapselsackes an dessen Peripherie auf einem Kreis anbringbar sind, dessen Normale die optische Achse des Auges ist,
**dadurch gekennzeichnet, dass** das system ferner versehen ist mit
• äußeren weiteren Permanentmagneten, die an der Sklera im Äußeren oder Inneren des Auges auf einem Kreis befestigt werden können, dessen Normale die optische Achse des Auges ist,
• einer geometrischen Anordnung und Polarisierung der Magnete, die so wählbar ist, daß innere und äußere Magnete sich abstoßen, wodurch eine resultierende Kraft nach vorne auf den Kapselsack ausgeübt wird, die diesen nach vorne verschiebt, wenn sich der Ziliarmuskel kontrahiert.

2. Akkommodatives Intraokularlinsensystem nach Anspruch 1, **gekennzeichnet dadurch, dass** die inneren Magnete in einem Kapselspannring integriert sind.

3. Akkommodatives Intraokularlinsensystem nach Anspruch 1, **gekennzeichnet dadurch, dass** im Bereich der inneren Magnete Hohlräume vorgesehen sind, um die Dichte des intraokularen Implantates zu reduzieren.

4. Akkommodatives Intraokularlinsensystem nach Anspruch 1, **gekennzeichnet dadurch, dass** die inneren Magnete in die Haptik der Intraokularlinse integriert sind.

5. Akkommodatives Intraokularlinsensystem nach Anspruch 1, **gekennzeichnet dadurch, dass** die äußeren Magnete Ösen, Kerben oder bereits befestigte Fäden aufweisen, die das Positionieren und Annähen erleichtern.

## Claims

1. Accomodative intraocular lens system with
• an intraocular lens in known technology, insertable into the capsular bag,
• inner permanent magnets which can be fitted inside the capsular bag at its periphery in a circle, the normal of which is the optical axis of the eye, **characterized by** the fact that the system further contains
• outer permanent magnets which can be fixed to the sclera outside or inside of the eye in a circle, the normal of which is the optical axis of the eye,
• a geometrical arrangement and a polarisation of the magnets which is selectable in such a manner that the inner and outer magnets repel one another, and that the resulting force pushes the capsular bag in anterior direction when the ciliary muscle contracts.

2. Accommodative intraocular lens system in accordance with claim 1, **characterized by** the fact that the inner magnets are integrated into a capsular tension ring.

3. Accommodative intraocular lens system in accordance with claim 1, **characterized by** the fact that hollow bodies are fixed to the inner magnets in order to reduce the density of the intraocular implant.

4. Accommodative intraocular lens system in accordance with claim 1, **characterized by** the fact that the inner magnets are integrated into the haptics of the intraocular lens.

5. Accommodative intraocular lens system in accordance with claim 1, **characterized by** the fact that eyelets, notches or suture material are fixed to the outer magnets in order to facilitate positioning and suturing.

## Revendications

1. Système correctif intra-oculaire à lentille comprenant
• une lentille intra-oculaire selon une technique connue et destinée à être insérée à l'intérieur du sac capsulaire,
• des aimants intérieurs permanents, à fixer à l'intérieur, à la périphérie, du sac capsulaire selon un cercle dont la normale est l'axe optique de l'oeil, **caractérisés en ce que** le système comporte également
• d'autres aimants extérieurs qui peuvent être fixés à la sclère à l'extérieur ou à l'intérieur de l'oeil selon un cercle dont la normale est l'axe optique de l'oeil,
• un placement géométrique et une polarisation des aimants qui peut être choisie de sorte que les aimants internes et externes se repoussent et que la force en résultant s'exerce vers l'avant, sur le sac capsulaire, et le déplace vers l'avant lorsque le muscle ciliaire se contracte.

2. Système correctif intra-oculaire à lentille selon la revendication 1, **caractérisé en ce que** les aimants intérieurs sont intégrés dans un anneau de tension capsulaire.

3. Système correctif intra-oculaire à lentille selon la revendication 1, **caractérisé en ce que**, dans la zone des aimants intérieurs, des vides sont prévus afin de réduire la densité de l'implant intra-oculaire.

4. Système correctif intra-oculaire à lentille selon la revendication 1, **caractérisé en ce que** les aimants intérieurs sont intégrés dans le système haptique de la lentille intra-oculaire.

5. Système correctif intra-oculaire à lentille selon la revendication 1, **caractérisé en ce que** les aimants extérieurs présentent des oeillets, encoches ou des fils pré-fixés qui facilitent le positionnement et la fixation par suture.
